(19) Europäisches Patentamt
European Patent Office
Office européen des brevets

(11) Numéro de publication: **0 267 105 B1**

# (12) FASCICULE DE BREVET EUROPEEN

(45) Date de publication de fascicule du brevet: **12.08.92**

(51) Int. Cl.[5]: **C07C 251/40**, C07D 213/64, A01N 53/00, A23K 1/16

(21) Numéro de dépôt: **87402440.9**

(22) Date de dépôt: **29.10.87**

## (54) Nouvelles oximes éthyléniques dérivés d'esters de l'acide cyclopropane carboxylique, leur procédé et les intermédiaires de préparation et leur application à la lutte contre les parasites.

(30) Priorité: **30.10.86 FR 8615124**

(43) Date de publication de la demande:
**11.05.88 Bulletin 88/19**

(45) Mention de la délivrance du brevet:
**12.08.92 Bulletin 92/33**

(84) Etats contractants désignés:
**CH DE GB IT LI NL**

(56) Documents cités:

**CHEMICAL ABSTRACTS, vol. 87, no. 1, 4.juillet 1977, page 108, no. 1139y, Columbus, Ohio, US; & JP-A-76125739 (SUMITOMO CHEMICAL CO. LTD.) 02.11.1976 & CHEMICAL ABSTRACTS TENTH COLLECTIVE INDEX, vol. 86-95, 1977-1981, American Chemical Society, US**

(73) Titulaire: **ROUSSEL-UCLAF**
**35, Boulevard des Invalides**
**F-75007 Paris(FR)**

(72) Inventeur: **Tessier, Jean**
**30, rue Jean Moulin**
**F-94300 Vincennes(FR)**
Inventeur: **Demoute, Jean-Pierre**
**249bis, rue de Rosny**
**F-Montreuil-Sous-Bois(FR)**
Inventeur: **Cadiergue, Joseph**
**6, rue Jules Princet**
**F-93600 Aulnay-Sous-Bois(FR)**

(74) Mandataire: **Tonnellier, Marie-José et al**
**Département des Brevets ROUSSEL UCLAF**
**B.P. no 9**
**F-93230 Romainville(FR)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention concerne de nouvelles oximes éthyléniques dérivés d'esters de l'acide cyclopropanecarboxylique, leur procédé et les intermédiaires de préparation et leur application à la lutte contre les parasites.

On connait par la demande de brevet japonais JP-A-51125739 les aldoximes dérivés des esters de l'acide 2,2-diméthyl (3-oxo 1-propényl) cyclopropanecarboxylique.

La présente invention a pour objet sous toutes les formes stéréoisomères possibles ainsi que les mélanges de ces stéréoisomères, les composés répondant à la formule (I) :

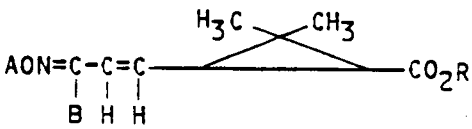

dans laquelle A représente :

- ou bien un atome d'hydrogène,
- ou bien un radical méthyle

B représente un radical méthyl

- soit un radical alcoyle linéaire, saturé ou insaturé, éventuellement substitué par un ou plusieurs atomes d'halogène, renfermant jusqu'à 18 atomes de carbone,
- soit un radical

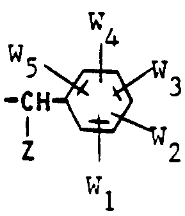

dans lequel Z représente un atome d'hydrogène, un radical alcoyle, alcényle ou alcynyle renfermant jusqu'à 18 atomes de carbone ou un radical cyano, $W_1$, $W_2$, $W_3$, $W_4$ et $W_5$, identiques ou différents, représentent un atome d'hydrogène un radical alcoyle, alcényle ou alcynyle, renfermant jusqu'à 18 atomes de carbone ou un radical cyano, un radical $CF_3$, $NO_2$, $O\text{-}R'_1$, $R'_1$ représentant un radical alcoyle, alcényle ou alcynyle renfermant jusqu'à 8 atomes de carbone, un atome d'halogène, un radical $CO_2\text{-}R'_2$ ou

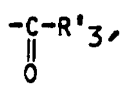

$R'_2$ et $R'_3$ représentant un radical alcoyle renfermant jusqu'à 18 atomes de carbone,

- soit un groupement

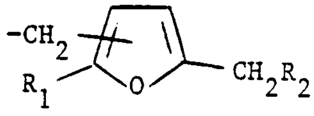

dans lequel $R_1$ représente un atome d'hydrogène ou un radical méthyle et le substituant $R_2$ un aryle monocyclique ou un groupement $-CH_2\text{-}C{\equiv}CH$ et notamment un groupement 5-benzyl 3-furyl méthyle,

- soit un groupement

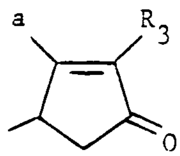

dans lequel a représente un atome d'hydrogène ou un radical méthyle et $R_3$ représente un radical organique aliphatique comportant de 2 à 6 atomes de carbone et une ou plusieurs insaturations carbone-carbone et notamment le radical $-CH_2-CH=CH_2$, $-CH_2-CH=CH-CH_3$, $-CH_2-CH=CH-CH=CH_2$, $-CH_2-CH=CH-CH_2-CH_3$, $-CH_2-C\equiv CH$,

- soit un groupement

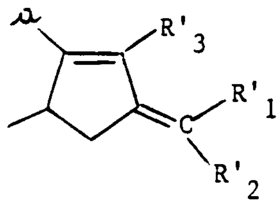

dans lequel a représente un atome d'hydrogène ou un radical méthyle, $R_3$ conserve la même signification que précédemment, $R'_1$ et $R'_2$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

- soit un groupement

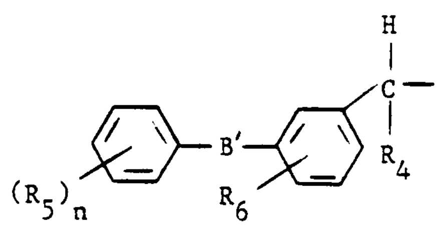

dans lequel B' représente un atome d'oxygène ou de soufre, un groupement

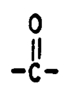

ou $-CH_2-$ ou un groupement sulfoxyde ou un groupement sulfone et $R_4$ représente un atome d'hydrogène, un radical $-C\equiv N$, un radical méthyle, un radical $-CONH_2$, un radical $-CSNH_2$ ou un radical $-C\equiv CH$, $R_5$ et $R_6$, identiques ou différents, représentent un atome d'hydrogène, un atome d'halogène ou un radical méthyle et n représente un nombre égal à 0, 1 ou 2, et notamment le groupement 3-phénoxybenzyle, alpha-cyano 3-phénoxy benzyle, alpha-éthynyl 3-phénoxy benzyle, 3-benzoyl benzyle, 1-(3-phénoxy phényl) éthyle ou alpha-thioamido 3-phénoxy benzyle,

- soit un groupement

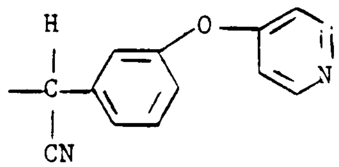

- soit un groupement

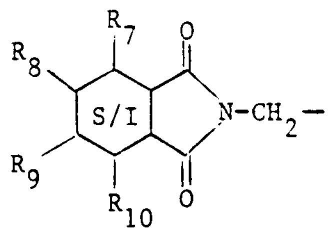

dans lequel les substituants $R_7$, $R_8$, $R_9$, $R_{10}$ représentent un atome d'hydrogène, un atome de chlore ou un radical méthyle et dans lequel S/I symbolise un cycle aromatique ou un cycle analogue déhydro ou tétrahydro,

- soit un groupement

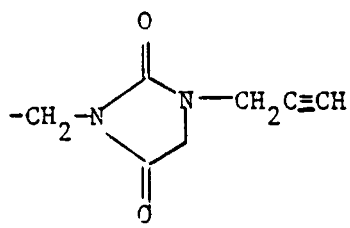

- soit un groupement

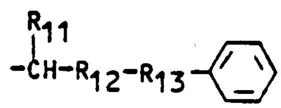

dans lequel $R_{11}$ représente un atome d'hydrogène ou un radical CN, $R_{13}$ représente un radical $-CH_2-$ ou un atome d'oxygène, $R_{12}$ représente un radical thiazolyle ou thiadiazolyle dont la liaison avec

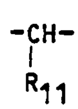

peut se trouver à l'une quelconque des positions disponibles, $R_{13}$ étant lié à $R_{12}$ par l'atome de carbone compris entre l'atome de soufre et un atome d'azote,

- soit un groupement

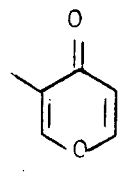

- soit un groupement

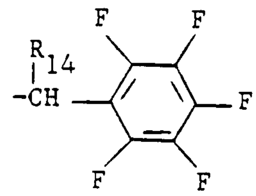

dans lequel $R_{14}$ représente un atome d'hydrogène ou un radical CN, ou un radical éthynyle,

- soit un groupement

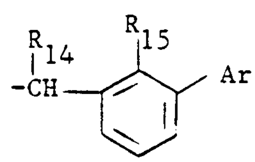

dans lequel $R_{14}$ représente un atome d'hydrogène, un radical méthyle, éthynyle ou cyano et $R_{15}$, différent, représente un atome d'hydrogène, de fluor ou de brome et Ar représente un radical aryle renfermant jusqu'à 14 atomes de carbone,

- soit un groupement

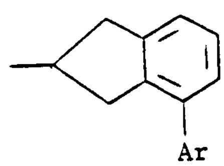

ou

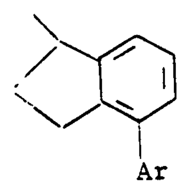

dans lequel Ar représente un radical aryle renfermant jusqu'à 14 atomes de carbone,

- soit un groupement

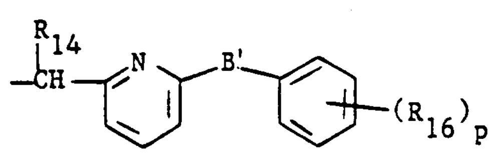

dans lequel $R_{14}$ est défini comme ci-dessus, chacun des $R_{16}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoylsulfonyle renfermant de 1 à 4 atomes de carbone, trifluorométhyle, 3,4-méthylènedioxy, chloro, fluoro ou bromo, p représente un nombre égal à 0, 1 ou 2 et B' représente un atome d'oxygène ou un atome de soufre,

- soit un radical

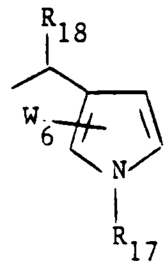

dans lequel $R_{17}$ représente un radical alcoyle insaturé renfermant jusqu'à 8 atomes de carbone et $W_6$ représente un atome d'hydrogène, un atome d'halogène, un radical alcoyle renfermant jusqu'à 18 atomes de carbone, un radical aryle renfermant jusqu'à 14 atomes de carbone, un radical aralcoyle renfermant jusqu'à 18 atomes de carbone, un radical cyano, un radical $CF_3$, un radical $CO_2$-alcoyle renfermant jusqu'à 8 atomes de carbone, un radical $NO_2$, un radical alcoxyle renfermant jusqu'à 8 atomes de carbone, $R_{18}$ représente un atome d'hydrogène, un radical -C≡N, un radical méthyle, un radical $CONH_2$, un radical $CSNH_2$ ou un radical -C≡CH.

Les composés de formule I peuvent posséder une copule cyclopropanique de structure cis trans, de structure cis ou de structure trans.

L'invention a plus particulièrement pour objet les composés de formule I dans lesquels la copule cyclopropanique est de structure 1R,cis ou 1R,trans.

Parmi les valeurs de A préférées, on peut citer les radicaux suivants :

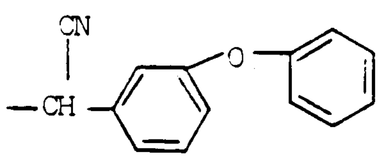

,

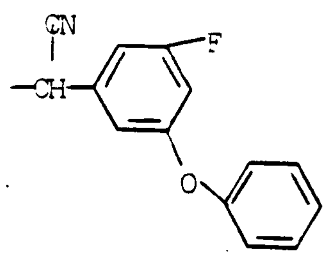

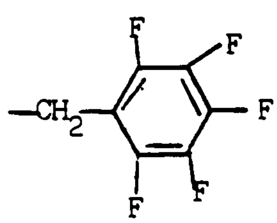

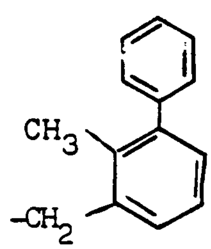

ou

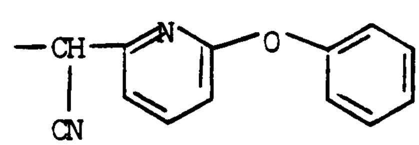

Comme valeurs préférées de A on peut citer le radical méthyle.

L'invention a tout spécialement pour objet les produits dont la préparation est donnée plus loin dans la partie expérimentale.

L'invention a également pour objet un procédé de préparation des composés de formule I caractérisé en ce que l'on soumet un acide de formule (II) :

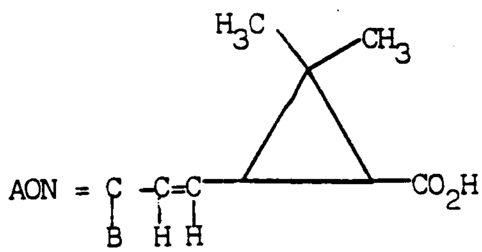

(II)

son chlorure d'acide ou un anhydride de cet acide dans laquelle A et B conservent leur signification précédente à l'action d'un alcool de formule ROH dans laquelle R conserve sa signification précédente pour obtenir le composé de formule (I) correspondant, et sépare si désiré, les différents stéréoisomères éventuellement obtenus.

L'estérification peut être réalisée, selon les procédés classiques d'estérification, notamment par estérification de l'acide par l'alcool en présence d'un carbodiimide avec ou sans base.

Les différents stéréoisomères peuvent être séparés par chromatographie.

Les produits de formule (II) sont des produits nouveaux et sont en eux-mêmes un des objets de la présente invention : ils peuvent être obtenus en faisant réagir, selon le réaction de Wittig, un composé de formule (III) :

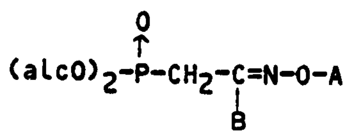

(III)

A et B conservant leur signification précédente et alc représentant un radical alcoyle renfermant de 1 à 4 atomes de carbone, en présence d'une base forte avec un acide de formule (IV)

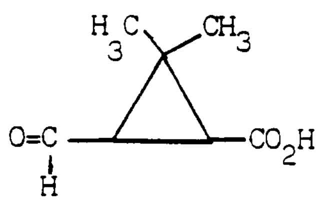

(IV)

sous la forme ouverte ou cyclisée (c'est-à-dire sous forme de lactone) hémiacétalique ou un dérivé de cet acide, pour obtenir le composé de formule II correspondant.

La base forte utilisée est de préférence un alcoolate alcalin, par exemple le terbutylate de sodium ou de potassium.

Les phosphonates utilisés comme produits de départ sont préparés selon le schéma suivant :

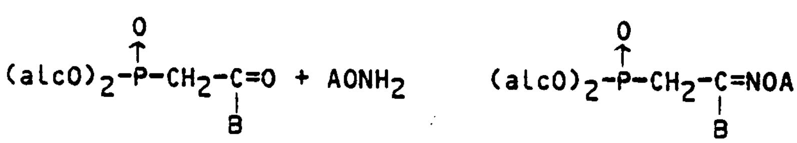

A et B conservant leur signification précédente.

Des exemples de préparation sont donnés plus loin dans la partie expérimentale.

Il va de soi que les produits de formule I peuvent également être préparés selon des procédés équivalents au procédé décrit ci-dessus, c'est ainsi que l'on peut préparer d'abord le composé de formule (V) :

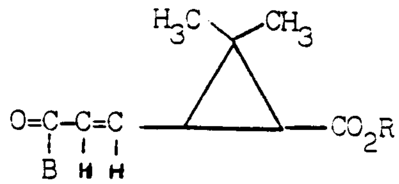

(V)

B et R conservant leur signification précédente, puis fait réagir ce composé de formule (V) avec un composé de formule (IV) :

$AONH_2$ (VI)

dans laquelle A a la signification indiquée précédemment pour obtenir le composé de formule (I) correspondant. Dans un mode de réalisation préféré, la condensation entre les produits de formule (V) et un sel du produit de formule (VI) a lieu en présence d'une base comme la pyridine.

Les composés de formule I présentent d'intéressantes propriétés qui permettent leur utilisation dans la lutte contre les parasites. Il peut s'agir, par exemple, de la lutte contre les parasites des végétaux, les parasites des locaux et les parasites des animaux à sang chaud.

Les produits de formule I présentent en particulier de remarquables propriétés d'abattage sur mouches.

C'est ainsi que l'on peut utiliser les produits de l'invention pour lutter contre les insectes, les nématodes et les acariens parasites des végétaux et des animaux.

L'invention a donc pour objet l'application des composés de formule I à la lutte contre les parasites des végétaux et les parasites des locaux.

Les produits de formule I peuvent donc être utilisés notamment pour lutter contre les insectes dans le domaine agricole, pour lutter, par exemple, contre les pucerons, les larves de lépidoptères et les coléoptères. Ils sont utilisés à des doses comprises entre 10 et 300 g de matière active à l'hectare.

Les produits de formule I peuvent également être utilisés pour lutter contre les insectes dans les locaux, pour lutter notamment contre les mouches, les moustiques et les blattes.

Les produits de formule I peuvent aussi être utilisés pour lutter contre les acariens et les nématodes parasites des végétaux.

Les composés de formule I peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Ambliyomnia et ceux de l'espèce Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule I définis ci-dessus.

L'invention a notamment pour objet les compositions insecticides renfermant comme principe actif au moins l'un des produits définis ci-dessus.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent en général, un véhicule et/ou un agent tensioactif non ionique, assurant en outre une dispersion uniforme des substances constitutives du mélange. Le véhicule utilisé peut être un liquide tel que l'eau, l'alcool, les hydrocarbures ou autres solvants organiques, une huile minérale, animale ou végétale, une poudre telle que le talc, les argiles, les silicates, le kiesselguhr ou un solide combustible.

Les compositions insecticides selon l'invention contiennent de préférence de 0,005% à 10% en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées pour la partie non active, d'un serpentin insecticide (ou coil) combustible, ou encore d'un substrat fibreux incombustible. Dans

ce dernier cas, le fumigant obtenu après incorporation de la matière active est placé sur un appareil chauffant tel qu'un électromosquito destroyer.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin), amidon et poudre de coque de noix de coco.

La dose de matière active peut alors être, par exemple, de 0,03 à 1% en poids.

Dans le cas où l'on utilise un support fibreux incombustible, la dose de matière active peut alors être, par exemple de 0,03 à 95% en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est de préférence, de 0,03 à 95% en poids.

Les compositions insecticides selon l'invention, comme les compositions acaricides et nématicides peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables pour pulvérisation foliaire, contenant de 1 à 80% ou des liquides pour pulvérisation foliaire contenant de 1 à 500 g/l de principe actif. On peut également employer des poudres pour poudrage foliaire contenant de 0,05 à 3% de matière active.

Pour l'usage nématicide, on utilise de préférence des liquides pour traitement des sols contenant de 300 à 500 g/l de principe actif.

Les composés acaricides et nématicides selon l'invention sont utilisés, de préférence à des doses comprises entre 1 et 100 g de matière active à l'hectare.

Pour exalter l'activité biologique des produits de l'invention, on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo [2,2-1] 5-heptène 2,3-dicarboximide, ou le pipéronyl-bis-2-(2'-n-butoxy éthoxy) éthylacétal (ou tropital).

Lorsqu'il s'agit de lutter contre les acariens parasites des animaux, on incorpore très souvent les produits de l'invention dans des compositions alimentaires en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple des farines de poissons, des acides aminés de synthèse, des sels minéraux, des vitamines et des antioxydants.

L'invention a donc ainsi pour objet les compositions alimentaires définies ci-dessus.

Les composés de formule I présentent une excellente tolérance générale, et l'invention a donc également pour objet les produits de formule I pour lutter notamment contre les tiques et les gales chez l'homme et l'animal.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on", ils peuvent être également administrés par voie digestive ou parentérale.

L'invention a également pour objet les associations douées d'activité insecticide, acaricide ou nématicide, caractérisées en ce qu'elles contiennent comme matière active, d'une part un au moins des composés de formule générale I, et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcools 3-phénoxy benzylique et d'alcools alpha-cyano 3-phénoxy benzyliques des acides chrysanthémiques, par les esters d'alcools 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools alpha-cyano 3-phénoxy benzyliques des acides 2,2-diméthyl 3-(2,2-dichlorovinyl) cyclopropane-1-carboxyliques, par les esters d'alcools alpha-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimidoiméthylique, d'alcool 5-benzyl 3-furyl méthylique, d'alcool 3-phénoxy benzylique et d'alcools alpha-cyano 3-phénoxy benzyliques des acides 2,2-diméthyl 3-(1,2,2,2,-tétrahaloéthyl) cyclopropane -1-carboxyliques, dans lesquels "halo" représente un atome de fluor, de chlore ou de brome, étant

entendu que les composés I peuvent exister sous toutes leurs formes stéréoisomères possibles de même que les copules acides et alcools des esters pyréthrinoïdes ci-dessus.

Les associations selon l'invention présentent notamment l'intérêt soit de permettre de combattre, par la polyvalence de leur action, une gamme de parasites plus étendue, soit de manifester, dans certains cas, un effet de synergie.

Les exemples suivants illustrent l'invention sans toutefois la limiter.

Exemple 1 :

1R trans 3-[delta Z, 3-(delta Z méthoxyimino) 1-buténÿl] 2,2-diméthyl cyclopropane carboxylate d'S cyano 1-(3-phénoxyphényl) méthyle, ainsi que les isomères 3-[delta Z, 3-(delta E méthoxyimino) 1-butényl], 3-[delta E,3-(delta E, méthoxyimino) 1-butényl] et 3-[delta E, 3-(delta Z méthoxyimino) 1-butényl] correspondants.

On refroidit à O¤C une solution renfermant 11,4 g d'acide 1R,trans 3-[3-méthoxyimino 1-buténylI 2,2-diméthyl cyclopropane carboxylique (mélange des 4 isomères E et Z au niveau des doubles liaisons), 11,55 g de S phénocyanol, 120 mg de 4-diméthylaminopyridine et 60 cm3 de chlorure de méthylène. On introduit à O¤C 12,2 g de dicyclohexylcarbodiimide et 60 cm3 de chlorure de méthylène. On laisse revenir à la température ambiante et filtre. Après évaporation du solvant, on obtient 25 g de produit brut que l'on chromatographie successivement sur silice en éluant par le mélange MeOtBu-hexane (2-8) puis sur silice en éluant par un mélange chloroforme-hexane-éther isopropylique (2-7-1). On obtient ainsi les 4 isomères recherchés.

Exemple 2 :

1R,cis 3-[delta E, 3-(delta E méthoxyimino) 1-butényl] 2,2-diméthyl cyclopropane carboxylate d'S cyano 1-(3-phénoxyphényl) méthyle et les isomères 3-[delta E, 3-(delta Z-méthoxyimino) 1-butényl 3-[delta Z, 3-(delta E-méthoxyimino) 1-butényl, et 3-[delta 2, 3-(delta Z méthoxyimino) 1-butényl] correspondants.

En opérant comme à l'exemple 1 à partir de l'acide 1R,cis 3-[3-méthoxyimino 1-butényl] 2,2-diméthyl cyclopropane carboxylique (mélange des quatre isomères E et Z au niveau des deux doubles liaisons et du S phénocyanol, on obtient les 4 isomères recherchés :

isomère delta E, delta E /alpha/$_D$ = + 23,5¤ ± 1¤ (c = 0,8% $CHCl_3$)
isomère delta E, delta Z /alpha/$_D$ = + 24,5¤ ± 2¤ (c = 0,5% $CHCl_3$)
isomère delta Z, delta E /alpha/$_D$ = + 73¤ ± 1,5¤ (c = 1,2% $CHCl_3$)

Préparation 1 :

Acide 1R,cis 3-[delta E, delta Z, 3-(delta E, delta Z-méthoxyimino) 1-butényl] 2,2-diméthyl cyclopropane carboxylique.

En opérant comme à la préparation 1, à partir de 2-méthoxyimino propylphosphonate de diméthyle et de lactone de l'acide 2,2-diméthyl 3-formyl cyclopropane carboxylique, on obtient le produit recherché.

Le produit de départ utilisé à savoir le 2-méthoxyimino propyl phosphonate de diméthyle a été préparé comme suit. On ajoute à 15¤C, 14,5g de chlorhydrate de méthylhydroxylamine et 40 cm3 d'eau dans une solution renfermant 22,2 g de béta-cétophosphonate de diméthyle

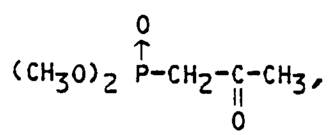

25 cm3 de pyridine et 235 cm3 de dioxane. On agite pendant une heure à la température ambiante. On évapore à sec. On ajoute 250 cm3 d'une solution aqueuse d'acide chlorhydrique N glacée et 250 cm3 de chlorure de méthylène. On décante, extrait la phase aqueuse au chlorure de méthylène. On sèche les phases organiques. On évapore sous pression réduite et obtient 24,8 g du produit brut que l'on chromatographie sur silice en éluant par le mélange chlorure de méthylène acétone (1/1). On obtient ainsi 20,8g du produit recherché.

Préparation 2 :

Acide 1R,trans 3-[delta E, delta Z, 3-(delta E, delta Z-méthoxyimino) 1-butényl] 2,2-diméthyl cyclopropane carboxylique.

En opérant comme à la préparation 1, à partir de 2-méthoxyiminopropyl phosphonate de diméthyle et d'acide 1 R,trans 2,2-diméthyl 3-formylcyclopropane carboxylique, on obtient le produit recherché.

Exemple 14 :

Préparation d'une composition fumigène.

On mélange d'une façon homogène :

| | |
|---|---|
| - Produit de l'exemple 1, isomère delta E, delta E | 0,25 g |
| - Poudre de tabu | 25,00 g |
| - Poudre de feuille de cèdre | 40,00 g |
| - Poudre de bois de pin | 33,75 g |
| - Vert brillant | 0,5 g |
| - p-nitrophénol | 0,5 g |

**ETUDE BIOLOGIOUE DES COMPOSES DE L'INVENTION.**

A) Etude de l'effet d'abattage sur mouche domestique.

Les insectes tests sont des mouches domestiques femelles âgées de 4 jours. On opère par pulvérisation directe à la concentration de 0,1 g/l en chambre de Kearns et March en utilisant comme solvant un mélange d'acétone (5%) et d'Isopar L (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.

Les résultats expérimentaux obtenus sont résumés dans le tableau suivant :

| Composés | | | KT 50 en mn |
|---|---|---|---|
| | C = N | C = C | |
| Exemple 1 | Z | Z | 5,1 |
| Exemple 1 | E | Z | 2,48 |
| Exemple 2 | Z | Z | 3,5 |

Conclusion :
Les produits présentent un pouvoir d'abattage très intéressant.

B) Etude de l'effet létal sur larves de Spodoptera Littoralis.

Les essais sont effectués par application topique d'une solution acétonique à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24¤C et 65% d'humidité relative. Après traitement les individus sont placés sur un milieu nutritif artificiel (milieu de Poitout).

On effectue le contrôle des mortalités 48 heures après traitement.

Les résultats expérimentaux obtenus sont résumés dans le tableau suivant :

| Composé | | | DL 50 en ng |
|---|---|---|---|
| | C = N | C = C | |
| Exemple 2 | Z | E | 6,4 |

Conclusion :
Le produit présent une bonne activité sur ce test.

**Revendications**

**1.** Sous toutes les formes stéréoisomères possibles, ainsi que les mélanges de ces stéréoisomères, les composés répondant à la formule I :

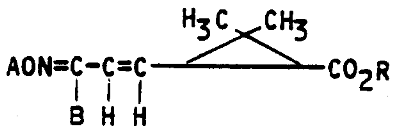

(I)

dans laquelle A représente :

- ou bien un atome d'hydrogène,
- ou bien un radical méthyle,

B représente un radical méthyle

R représente

- soit un radical alcoyle linéaire, saturé ou insaturé, éventuellement substitué par un ou plusieurs atomes d'halogène, renfermant jusqu'à 18 atomes de carbone,
- soit un radical

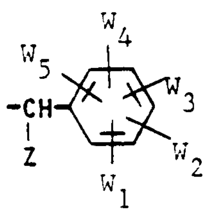

dans lequel Z représente un atome d'hydrogène, un radical alcoyle, alcényle ou alcynyle renfermant jusqu'à 18 atomes de carbone ou un radical cyano, $W_1$ et $W_2$, identiques ou différents, représentent un atome d'hydrogène, un radical alcoyle, alcényle ou alcynyle, renfermant jusqu'à 18 atomes de carbone ou un radical cyano, un radical $CF_3$, $NO_2$, O-R'$_1$, R'$_1$ représentant un radical alcoyle, alcényle ou alcynyle renfermant jusqu'à 8 atomes de carbone, un atome d'halogène, un radical $CO_2$-R'$_2$ ou

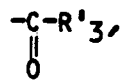

R'$_2$ et R'$_3$ représentant un radical alcoyle renfermant jusqu'à 18 atomes de carbone,

- soit un groupement

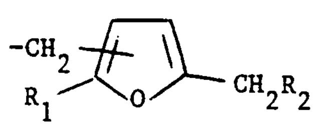

dans lequel $R_1$ représente un atome d'hydrogène ou un radical méthyle et le substituant $R_2$ un aryle monocyclique ou un groupement -$CH_2$-C≡CH et notamment un groupement 5-benzyl 3-furyl méthyle,

- soit un groupement

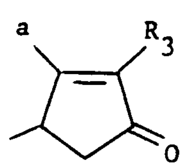

dans lequel a représente un atome d'hydrogène ou un radical méthyle et $R_3$ représente un radical organique aliphatique comportant de 2 à 6 atomes de carbone et une ou plusieurs insaturations carbone-carbone et notamment le radical $-CH_2-CH=CH_2$, $-CH_2-CH=CH-CH_3$, $-CH_2-CH=CH-CH=CH_2$, $-CH_2-CH=CH-CH_2-CH_3$, $-CH_2-C\equiv CH$,

- soit un groupement

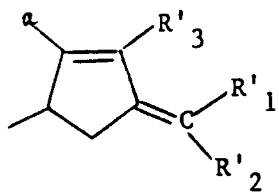

dans lequel a représente un atome d'hydrogène ou un radical méthyle, $R'_3$ conserve la même signification que précédemment, $R'_1$ et $R'_2$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

- soit un groupement

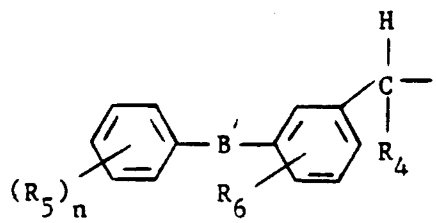

dans lequel B' représente un atome d'oxygène ou de soufre, un groupement

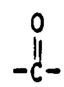

ou $-CH_2-$ ou un groupement sulfoxyde ou un groupement sulfone et $R_4$ représente un atome d'hydrogène, un radical $-C\equiv N$, un radical méthyle, un radical $-CONH_2$, un radical $-CSNH_2$ ou un radical $-C\equiv CH$, $R_5$ et $R_6$, identiques ou différents, représentent un atome d'hydrogène, un atome d'halogène ou un radical méthyle et n représente un nombre égal à 0, 1 ou 2, et notamment le groupement 3-phénoxybenzyle, alpha-cyano 3-phénoxy benzyle, alpha-éthynyl 3-phénoxy benzyle, 3-benzoyl benzyle, 1-(3-phénoxy phényl) éthyle ou alpha-thioamido 3-phénoxy benzyle,

- soit un groupement

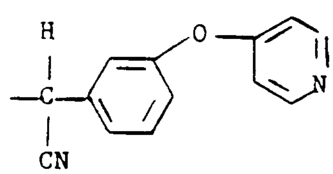

- soit un groupement

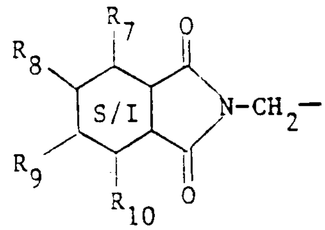

dans lequel les substituants $R_7$, $R_8$, $R_9$, $R_{10}$ représentent un atome d'hydrogène, un atome de chlore ou un radical méthyle et dans lequel S/I symbolise un cycle aromatique ou un cycle analogue déhydro ou tétrahydro,

- soit un groupement

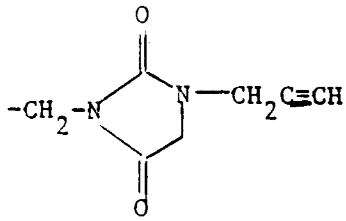

- soit un groupement

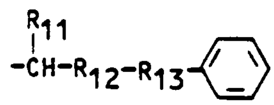

dans lequel $R_{11}$ représente un atome d'hydrogène ou un radical CN, $R_{13}$ représente un radical $-CH_2-$ ou un atome d'oxygène, $R_{12}$ représente un radical thiazolyle ou thiadiazolyle dont la liaison avec

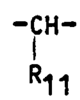

peut se trouver à l'une quelconque des positions disponibles, $R_{13}$ étant lié à $R_{12}$ par l'atome de carbone compris entre l'atome de soufre et un atome d'azote,

- soit un groupement

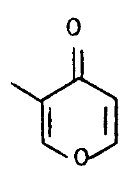

- soit un groupement

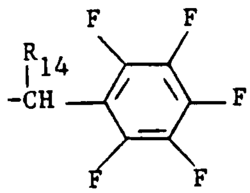

dans lequel $R_{14}$ représente un atome d'hydrogène ou un radical CN, ou un radical éthynyle,

- soit un groupement

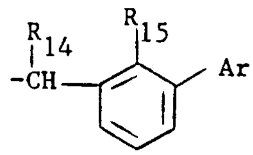

dans lequel $R_{14}$ représente un atome d'hydrogène, un radical méthyle, éthynyle ou cyano et $R_{15}$, différent, représente un atome d'hydrogène, de fluor ou de brome et Ar représente un radical aryle renfermant jusqu'à 14 atomes de carbone,

- soit un groupement

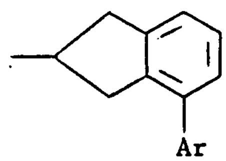

ou

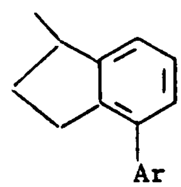

dans lequel Ar représente un radical aryle renfermant jusqu'à 14 atomes de carbone,

- soit un groupement

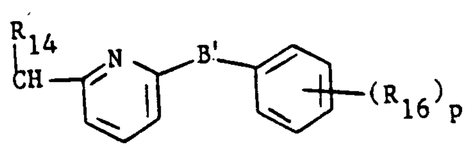

dans lequel $R_{14}$ est défini comme ci-dessus, chacun des $R_{16}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoylsulfonyle renfermant de 1 à 4 atomes de carbone, trifluorométhyle, 3,4-méthylènedioxy, chloro, fluoro ou bromo, p représente un nombre égal à 0, 1 ou 2 et B' représente un atome d'oxygène ou un atome de soufre,

- soit un radical

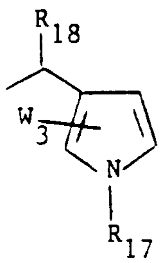

dans laquelle $R_{17}$ représente un radical alcoyle insaturé renfermant jusqu'à 8 atomes de carbone et $W_6$ représente un atome d'hydrogène, un atome d'halogène, un radical alcoyle renfermant jusqu'à 18 atomes de carbone, un radical aryle renfermant jusqu'à 14 atomes de carbone, un radical aralcoyle renfermant jusqu'à 18 atomes de carbone, un radical cyano, un radical $CF_3$, Un radical $CO_2$-alcoyle renfermant jusqu'à 8 atomes de carbone, un radical $NO_2$, un radical alcoxyle renfermant jusqu'à 8 atomes de carbone, $R_{18}$ représente un atome d'hydrogène, un radical -C≡N, un radical méthyle, un radical $CONH_2$, un radical $CSNH_2$ ou un radical -C≡CH.

**2.** Les composés de formule I tels que définis à la revendication 1 dans lesquels la copule cyclopropanique est de structure 1R cis ou 1R trans.

**3.** Les composés de formule I tels que définis à la revendication 1 dans lesquels R représente l'un des radicaux suivants :

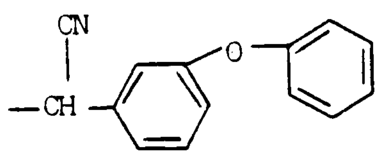

,

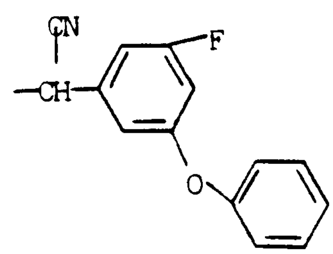

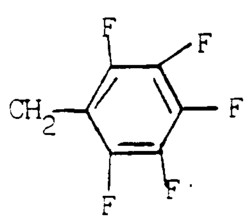

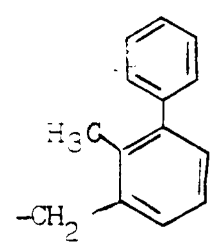

ou

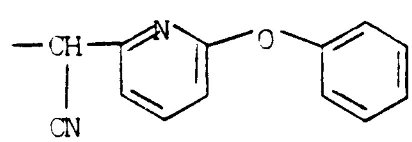

**4.** Les composés de formule I tels que définis à l'une quelconque des revendications 1 à 3 dans lesquels A représente un radical méthyle.

**5.** Les composés définis à la revendication 1 dont les noms suivent :
le 1R,cis 3-[delta Z,(3 delta Z, méthoxyimino) 1-propényl] 2,2diméthyl cyclopropane carboxylate du S cyano 1 (3-phénoxyphényl) méthyle. le 1R,cis 3-[delta Z,(3 delta E méthoxyimino 1-propényl] 2,2-diméthyl cyclopropane carboxylate d'S cyano 1-(3-phénoxyphényl) méthyle.

**6.** Procédé de préparation des composés de formule I tels que définis à l'une quelconque des revendications 1 à 5 caractérisé en ce que l'on soumet un acide de formule (II) :

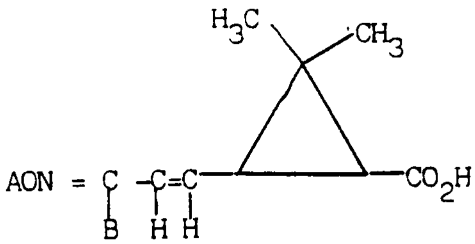

(II)

son chlorure d'acide ou ses anhydrides, dans laquelle A et B conservent leur signification précédente à l'action d'un alcool de formule ROH dans laquelle R conserve sa signification précédente pour obtenir le composé de formule (I) correspondant, et sépare si désiré, les différents stéréoisomères éventuellement obtenus.

**7.** Variante du procédé de préparation des composés de formule (I), selon la revendication 6, caractérisée en ce que l'on soumet un composé de formule (V) :

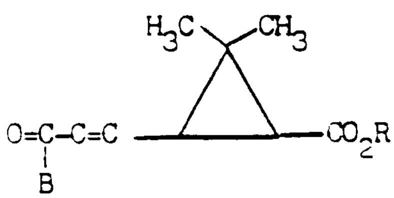

(V)

dans laquelle B et R sont définis comme dans la revendication 1, à l'action d'un composé de formule (VI) :

$AONH_2$ (VI)

dans laquelle A a la signification indiquée précédemment pour obtenir le composé de formule (I) correspondant.

**8.** A titre de produits intermédiaires, les acides de formule (II) ainsi que leur chlorure d'acide ou les anhydrides de ces acides, tels que définis à la revendication 6.

**9.** Application des composés de formule (I) tels que définis à l'une quelconque des revendications 1 à 5 à la lutte contre les parasites des végétaux, les parasites des locaux.

**10.** Les compositions destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 5.

**11.** Les compositions insecticides, caractérisées en ce qu'elles renferment comme principe actif au moins un composé défini à l'une quelconque des revendications 1 à 5.

**12.** Les compositions acaricides, caractérisées en ce qu'elles renferment comme principe actif au moins un composé défini à l'une quelconque des revendications 1 à 5.

**13.** Les compositions destinées à l'alimentation animale, caractérisées en ce qu'elles renferment comme principe actif un au moins des composés définis à l'une quelconque des revendications 1 à 5 associé à un aliment pour animal.

**14.** Associations douées d'activité insecticide, acaricide ou nématicide, caractérisées en ce qu'elles contiennent comme matière active, d'une part, un au moins des composés de formule générale (I), et, d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique,

d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidéne méthyl) cyclopropane-1-carboxyliques, par les esters d'alcool 3-phénoxy benzylique et d'alcools alpha-cyano 3-phénoxy benzyliques des acides 2,2- diméthyl 3-(2,2dichlorovinyl) cyclopropane-1-carboxyliques, par les esters d'alcools alpha-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques par les esters d'alléthrolones, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides 2,2-diméthyl 3-(1,2,2,2-tétrahaloéthyl) cyclopropane-1-carboxyliques, dans lesquels "halo" représente un atome de fluor, de chlore ou de brome, étant entendu que les composés I peuvent exister sous toutes leurs formes stéréoisomères possibles de même que les copules acides et alcools des esters pyréthrinoïdes ci-dessus.

## Claims

**1.** In all possible stereoisomer forms, as well as mixtures of these stereoisomers, the compounds corresponding to formula I:

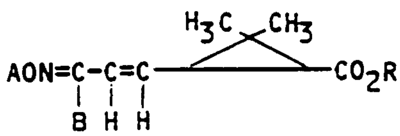

(I)

in which A represents:
- either a hydrogen atom,
- or a methyl radical,

B represents a methyl radical

R represents
- either a saturated or unsaturated linear alkyl radical, optionally substituted by one or more halogen atoms, containing up to 18 carbon atoms,
- or a

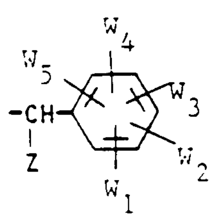

radical,
in which z represents a hydrogen atom, an alkyl, alkenyl or alkynyl radical containing up to 18 carbon atoms or a cyano radical, $W_1$ and $W_2$, identical or different, represent a hydrogen atom, an alkyl, alkenyl or alkynyl radical containing up to 18 carbon atoms or a cyano radical, a $CF_3$, $NO_2$, O-$R'_1$ radical, $R'_1$ representing an alkyl, alkenyl or alkynyl radical containing up to 8 carbon atoms, a halogen atom, a $CO_2$-$R'_2$ or

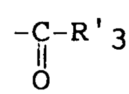

radical, $R'_2$ and $R'_3$ representing an alkyl radical containing up to 18 carbon atoms,
- or a

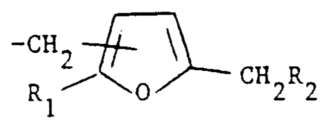

group in which $R_1$ represents a hydrogen atom or a methyl radical and the substituent $R_2$ a monocyclic aryl or a $-CH_2-C=CH$ group and in particular a 5-benzyl-3-furyl methyl group.

- or a

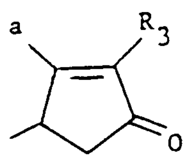

group in which a represents a hydrogen atom or a methyl radical and $R_3$ represents an organic aliphatic radical containing 2 to 6 carbon atoms and one or more carbon-carbon unsaturations and in particular the $-CH_2-CH=CH_2$, $-CH_2-CH=CH-CH_3$, $-CH_2-CH=CH-CH=CH_2$, $-CH_2-CH=CH-CH_2-CH_3$, $-CH_2-C\equiv CH$ groups,

- or a

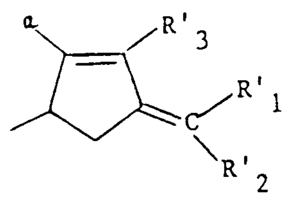

group in which a represents a hydrogen atom or a methyl radical, $R'_3$ retains the same meaning as previously, $R'_1$ and $R'_2$, identical or different, represent a hydrogen atom, a halogen atom, an alkyl radical containing 1 to 6 carbon atoms, an aryl radical containing 6 to 10 carbon atoms, an alkyloxycarbonyl group containing 2 to 5 carbon atoms, or a cyano group,

- or a

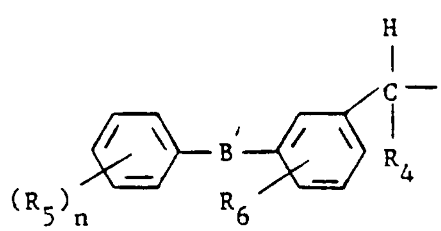

group in which B' represents an oxygen or sulphur atom, a

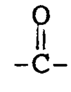

or $-CH_2-$ group or a sulphoxide group or a sulphone group and $R_4$ represents a hydrogen atom, a $-C\equiv N$ radical, a methyl radical, a $-CONH_2$ radical, a $-CSNH_2$ radical or a $-C\equiv CH$ radical, $R_5$ and

$R_6$, identical or different, represent a hydrogen atom, a halogen atom or a methyl radical and n represents a number equal to 0, 1 or 2, and in particular the following groups: 3-phenoxybenzyl, alpha-cyano-3-phenoxy benzyl, alpha-ethynyl-3-phenoxy benzyl, 3-benzoyl benzyl, 1-(3-phenoxy-phenyl) ethyl or alpha-thioamido-3-phenoxy benzyl,

- or a group

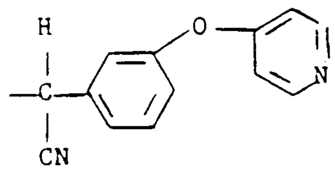

- or a group

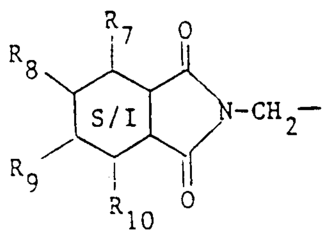

in which the $R_7$, $R_8$, $R_9$ $R_{10}$ substituents represent a hydrogen atom, a chlorine atom or a methyl radical and in which S/I symbolizes an aromatic ring or an analogous dehydro or tetrahydro ring,

- or a group

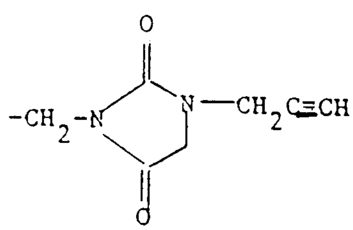

- or a group

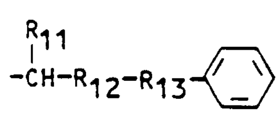

in which $R_{11}$ represents a hydrogen atom or a CN radical, $R_{13}$ represents a $-CH_2-$ radical or an oxygen atom, $R_{12}$ represents a thiazolyl or thiadiazolyl radical of which the bond with

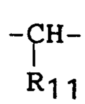

can be found in any one of the available positions, $R_{13}$ being linked to $R_{12}$ by a carbon atom

comprised between the sulphur atom and the nitrogen atom,

- <u>or</u> a group

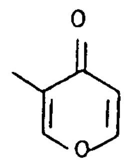

- <u>or</u> a group

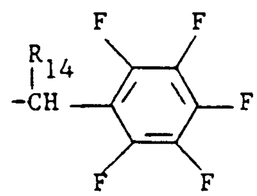

in which $R_{14}$ represents a hydrogen atom or a CN radical, or an ethynyl radical,

- <u>or</u> a group

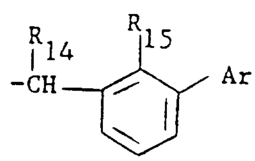

in which $R_{14}$ represents a hydrogen atom, a methyl, ethynyl or cyano radical and $R_{15}$, being different, represents a hydrogen, fluorine or bromine atom and Ar represents an aryl radical containing up to 14 carbon atoms,

- <u>or</u> a group

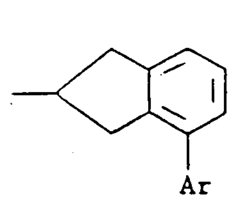

or

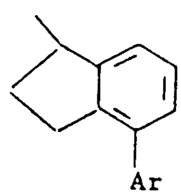

in which Ar represents an aryl radical containing up to 14 carbon atoms,

- <u>or</u> a group

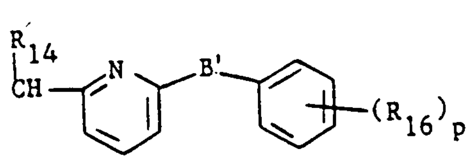

in which $R_{14}$ is defined as above, each of the $R_{16}$'s represents independently one of the following groups: alkyl containing 1 to 4 carbon atoms, alkoxy containing 1 to 4 carbon atoms, alkylthio

containing 1 to 4 carbon atoms, alkylsulphonyl containing 1 to 4 carbon atoms, trifluoromethyl, 3,4-methylenedioxy, chloro, fluoro or bromo, p represents a number equal to 0, 1 or 2 and B' represents an oxygen or sulphur atom,

- or a radical

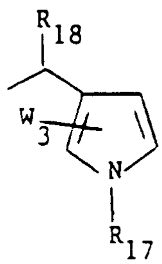

in which $R_{17}$ represents an unsaturated alkyl radical containing up to 8 carbon atoms and $W_6$ represents a hydrogen atom, a halogen atom, an alkyl radical containing up to 18 carbon atoms, an aryl radical containing up to 14 carbon atoms, an aralkyl radical containing up to 18 carbon atoms, a cyano radical, a $CF_3$ radical, a $CO_2$-alkyl radical containing up to 8 carbon atoms, an $NO_2$ radical, an alkoxyl radical containing up to 8 carbon atoms, $R_{18}$ represents a hydrogen atom, a -C=N radical, a methyl radical, a $CONH_2$ radical, a $CSNH_2$ radical or a -C≡CH radical.

**2.** The compounds of formula I as defined in claim 1 in which the cyclopropane copula is of 1R cis or 1R trans structure.

**3.** The compounds of formula I as defined in claim 1 in which R represents one of the following radicals:

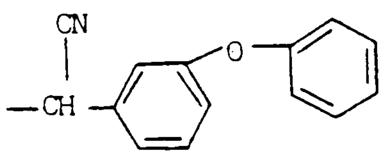

,

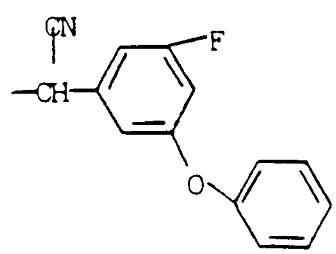

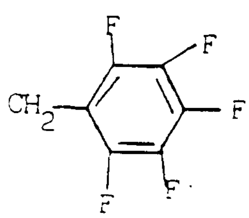

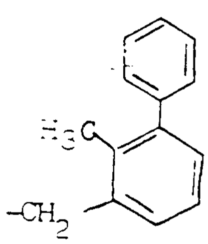

or

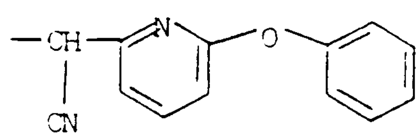

**4.** The compounds of formula I as defined in any one of claims 1 to 3 in which A represents a methyl radical.

**5.** The compounds defined in claim 1 the names of which follow:
1R,cis 3-[delta Z,(3 delta Z, methoxyimino)-1-propenyl]-2,2-dimethyl cyclopropane carboxylate of S cyano-1-(3-phenoxyphenyl) methyl.
1R,cis 3-[delta Z,(3 delta E methoxyimino)-1-propenyl]-2,2-dimethyl cyclopropane carboxylate of S cyano-1-(3-phenoxyphenyl) methyl.

**6.** Preparation process for the compounds of formula I as defined in any one of claims 1 to 5, characterized in that an acid of formula (II):

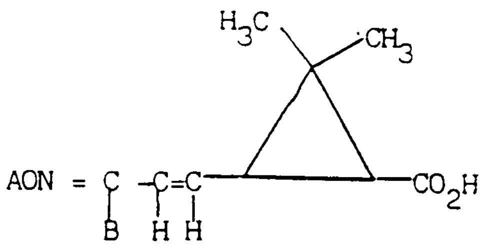

(II)

its acid chloride or its anhydrides, in which A and B retain their previous meaning, is subjected to the action of an alcohol of formula ROH in which R retains its previous meaning in order to obtain the corresponding compound of formula (I), and if desired the optionally obtained different stereoisomers are separated.

**7.** Variant of the preparation process for the compounds of formula (I), according to claim 6, characterized in that a compound of formula (V):

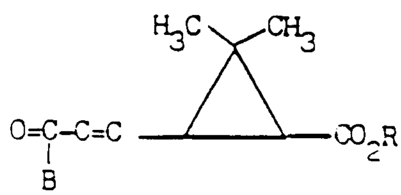

(V)

in which B and R are as defined in claim 1, is subjected to the action of a compound of formula (VI):

$AONH_2$ (VI)

in which A has the meaning indicated previously in order to obtain the corresponding compound of formula (I).

**8.** As intermediate products, the acids of formula (II) as well as their acid chloride or the anhydrides of these acids, as defined in claim 6.

**9.** The use of the compounds of formula (I) as defined in any one of claims 1 to 5 for combating parasites of vegetation and parasites of premises.

**10.** The compositions intended for combating parasites of vegetation, parasites of premises and parasites of warm-blooded animals, characterized in that they contain at least one compound defined in any one of claims 1 to 5 as active ingredient.

**11.** The insecticide compositions, characterized in that they contain at least one compound defined in any one of claims 1 to 5 as active ingredient.

**12.** The acaricide compositions, characterized in that they contain at least one compound defined in any one of claims 1 to 5 as active ingredient.

**13.** The compositions intended for animal feed, characterized in that they contain at least one of the compounds defined in any one of claims 1 to 5 as active ingredient combined with an animal feed.

**14.** Combinations endowed with insecticide, acaricide or nematicide activity characterized in that they contain as active ingredient, on the one hand at least one of the compounds of general formula (I) and on the other hand, at least one of the pyrethrinoid esters chosen from the group constituted by the esters of allethrones, of 3,4,5,6-tetrahydrophthalimidomethyl alcohol, of 5-benzyl-3-furyl methyl alcohol, of 3-phenoxybenzyl alcohol and of alpha-cyano-3-phenoxybenzylalcohols with chrysanthemic acids, by

the esters of 5-benzyl-3-furyl methyl alcohol with 2,2-dimethyl-3-(2-oxo-3-tetrahydrothiophenylidenemethyl)-cyclopropane-1-carboxylic acids, by the esters of 3-phenoxybenzyl alcohol and of alpha-cyano-3-phenoxybenzyl alcohols with 2,2-dimethyl-3-(2,2-dichlorovinyl)-cyclopropane-1-carboxylic acids, by the esters of alpha-cyano-3-phenoxy-benzyl alcohols with 2,2-dimethyl-3-(2,2-dibromovinyl)-cyclopropane-1-carboxylic acids, by the esters of 3-phenoxybenzyl alcohol with 2-parachlorophenyl-2-isopropyl acetic acids, by the esters of allethrolones, of 3,4,5,6-tetrahydrophthalimidomethyl alcohol, of 5-benzyl-3-furyl methyl alcohol, of 3-phenoxybenzyl alcohol, and of alpha-cyano-3-phenoxybenzyl alcohols with 2,2-dimethyl-3-(1,2,2,2-tetrahaloethyl)-cyclopropane-1-carboxylic acids, in which "halo" represents a fluorine, chlorine or bromine atom, it being understood that the compounds (I) can exist in all their possible stereoisomer forms as well as the acid and alcohol copulas of the above pyrethrinoid esters.

## Patentansprüche

**1.** In sämtlichen ihrer möglichen stereoisomeren Formen sowie als Gemische dieser Stereoisomeren die Verbindungen der Formel I

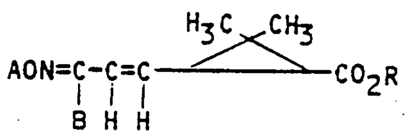

(I)

worin A bedeutet:
- entweder ein Wasserstoffatom
- oder einen Methylrest,

B bedeutet: einen Methylrest,
R bedeutet:
- entweder einen linearen, gesättigten oder ungesättigten, gegebenenfalls durch ein oder mehrere Halogenatome substituierten Alkylrest mit bis zu 18 Kohlenstoffatomen
- oder einen Rest

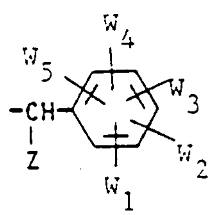

worin Z ein Wasserstoffatom, einen Alkyl-, Alkenyl- oder Alkinylrest mit bis zu 18 Kohlenstoffatomen oder eine Cyanogruppe bedeutet, $W_1$ und $W_2$, die gleich oder voneinander verschieden sind, für ein Wasserstoffatom, einen Alkyl-, Alkenyl- oder Alkinylrest mit bis zu 18 Kohlenstoffatomen oder eine Cyanogruppe, einen Rest $CF_3$, $NO_2$, O-$R'_1$ stehen, wobei $R'_1$ einen Alkyl-, Alkenyl- oder Alkinylrest mit bis zu 8 Kohlenstoffatomen, ein Halogenatom, eine Gruppe $CO_2$-$R'_2$ oder

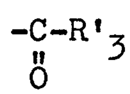

wiedergibt, in der $R'_2$ und $R'_3$ einen Alkylrest mit bis zu 18 Kohlenstoffatomen bedeuten,
- oder eine Gruppe

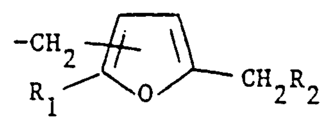

worin $R_1$ ein Wasserstoffatom oder eine Methylgruppe bedeutet und der Substituent $R_2$ ein monocyclischer Arylrest oder eine Gruppe $-CH_2-C\equiv CH$ und insbesondere eine 5-Benzyl-3-furylmethylgruppe ist,

- oder eine Gruppe

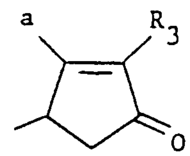

worin a für ein Wasserstoffatom oder einen Methylrest steht und $R_3$ einen aliphatischen, organischen Rest mit 2 bis 6 Kohlenstoffatomen und einer oder mehreren Kohlenstoff-Kohlenstoff-Unsättigungen und insbesondere den Rest $-CH_2-CH=CH_2$, $-CH_2-CH=CH-CH_3$, $-CH_2-CH=CH-CH=CH_2$, $-CH_2-CH=CH-CH_2-CH_3$, $-CH_2-C\equiv CH$ bedeutet,

- oder eine Gruppe

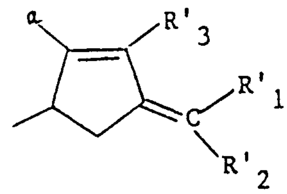

worin a ein Wasserstoffatom oder eine Methylgruppe bedeutet, $R'_3$ die vorstehend angegebene Bedeutung besitzt, $R'_1$ und $R'_2$, die gleich oder voneinander verschieden sind, ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen oder eine Cyanogruppe wiedergeben,

- oder eine Gruppe

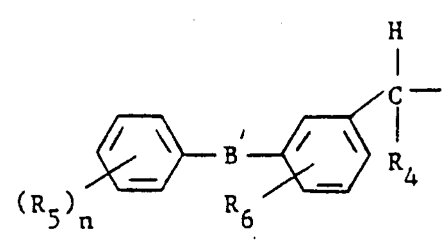

worin B' für ein Sauerstoff- oder Schwefelatom, eine Gruppe

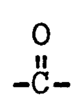

oder $-CH_2-$ oder eine Sulfoxidgruppe oder eine Sulfongruppe steht und $R_4$ ein Wasserstoffatom, einen Rest $-C\equiv N$, eine Methylgruppe, einen Rest $-CONH_2$, einen Rest $-CSNH_2$ oder einen Rest

-C≡CH bedeutet, $R_5$ und $R_6$, die gleich oder voneinander verschieden sind, für ein Wasserstoffatom, ein Halogenatom oder eine Methylgruppe stehen und n eine Zahl entsprechend 0, 1 oder 2 wiedergibt,und insbesondere die 3-Phenoxybenzyl-, α-Cyano-3-phenoxybenzyl-, α-Ethinyl-3-phenoxybenzyl-, 3-Benzoylbenzyl-, 1-(3-Phenoxphenyl)-ethyl- oder α-Thioamido-3-phenoxybenzylgruppe

- oder eine Gruppe

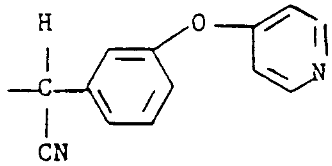

- oder eine Gruppe

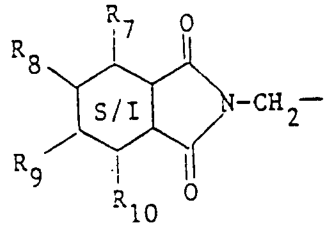

worin die Substituenten $R_7$, $R_8$, $R_9$, $R_{10}$ für ein Wasserstoffatom, ein Chloratom oder eine Methylgruppe stehen und worin S/I einen aromatischen Ring oder einen analogen Dehydro- oder Tetrahydroring wiedergibt,

- oder eine Gruppe

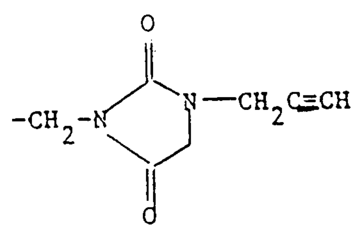

- oder eine Gruppe

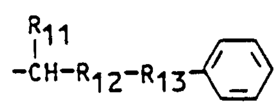

worin $R_{11}$ für ein Wasserstoffatom oder eine CN-Gruppe steht, $R_{13}$ einen Rest $-CH_2-$ oder ein Sauerstoffatom wiedergibt, $R_{12}$ einen Thiazolyl- oder Thiadiazolylrest bedeutet, dessen Bindung mit

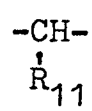

sich an irgendeiner der verfügbaren Positionen befinden kann, wobei $R_{13}$ an $R_{12}$ über ein

Kohlenstoffatom zwischen dem Schwefelatom und einem Stickstoffatom gebunden ist,

- oder eine Gruppe

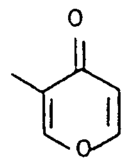

- oder eine Gruppe

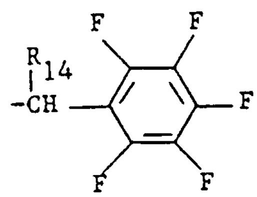

worin $R_{14}$ für ein Wasserstoffatom oder eine CN-Gruppe oder einen Ethinylrest steht,

- oder eine Gruppe

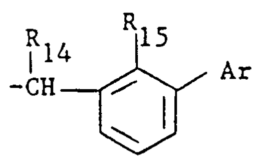

worin $R_{14}$ ein Wasserstoffatom, einen Methyl-, Ethinyl- oder Cyanorest bedeutet und $R_{15}$, verschieden, ein Wasserstoff-, Fluor- oder Bromatom bedeutet und Ar für einen Arylrest mit bis zu 14 Kohlenstoffatomen steht,

- oder eine Gruppe

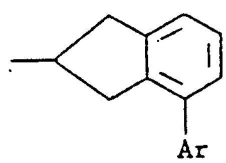

oder

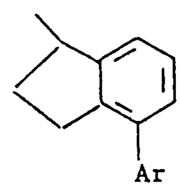

worin Ar einen Arylrest mit bis zu 14 Kohlenstoffatomen bedeutet,

- oder eine Gruppe

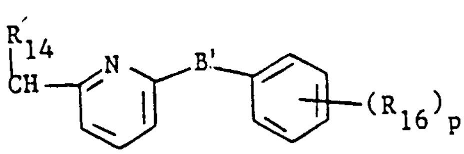

worin $R_{14}$ wie vorstehend definiert ist, ein jeder der Reste $R_{16}$ unabhängig eine Alkylgruppe mit 1 bis 4 Kohlenstoffatomen, eine Alkoxygruppe mit 1 bis 4 Kohlenstoffatomen, eine Alkylthiogruppe

mit 1 bis 4 Kohlenstoffatomen, eine Alkylsulfonylgruppe mit 1 bis 4 Kohlenstoffatomen, eine Trifluormethyl-, 3,4-Methylendioxygruppe, Chlor, Fluor oder Brom bedeutet, p eine Zahl entsprechend 0, 1 oder 2 wiedergibt und B' für ein Sauerstoffatom oder ein Schwefelatom steht,

- oder eine Gruppe

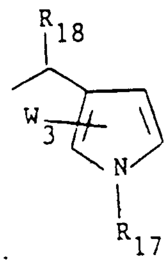

worin $R_{17}$ einen ungesättigten Alkylrest mit bis zu 8 Kohlenstoffatomen bedeutet und $W_6$ für ein Wasserstoffatom, ein Halogenatom, einen Alkylrest mit bis zu 18 Kohlenstoffatomen, einen Arylrest mit bis zu 14 Kohlenstoffatomen, einen Aralkylrest mit bis zu 18 Kohlenstoffatomen, eine Cyanogruppe, eine Gruppe $CF_3$, einen $CO_2$-alkylrest mit bis zu 8 Kohlenstoffatomen, eine Gruppe $NO_2$, eine Alkoxygruppe mit bis zu 8 Kohlenstoffatomen steht, $R_{18}$ ein Wasserstoffatom, einen Rest -C≡N, eine Methylgruppe, einen Rest $CONH_2$, einen Rest $CSNH_2$ oder einen Rest -C≡CH wiedergibt.

**2.** Verbindungen der Formel I gemäß Anspruch 1, worin die Cyclopropan-Verknüpfung die 1R,cis- oder IR,trans-Struktur aufweist.

**3.** Verbindungen der Formel I gemäß Anspruch 1, worin R für einen der folgenden Reste steht:

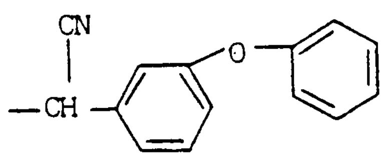

,

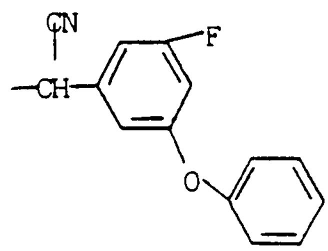

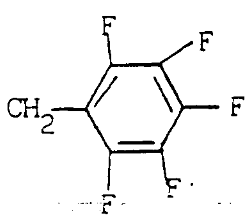

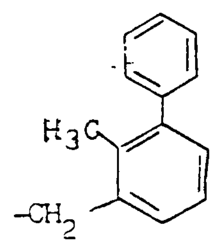

oder

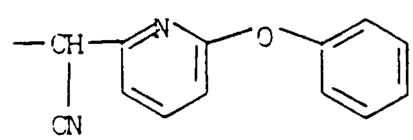

**4.** Verbindungen der Formel I gemäß einem der Ansprüche 1 bis 3, worin a eine Methylgruppe bedeutet.

**5.** Verbindungen gemäß Anspruch 1 mit den folgenden Bezeichnungen:
S-Cyano-1-(3-phenoxyphenyl)-methyl-1R,cis-3-[ΔZ-(3ΔZ-methoxyimino)-1-propenyl]-2,2-dimethylcyclopropancarboxylat,
S-Cyano-1-(3-phenoxyphenyl)-methyl-1R,cis-3-[ΔZ-(3ΔE-methoxyimino)-1-propenyl]-2,2-dimethylcyclopropancarboxylat.

**6.** Verfahren zur Herstellung der Verbindungen der Formel I, wie in einem der Ansprüche 1 bis 5 definiert, dadurch gekennzeichnet, daß man eine Säure der Formel (II)

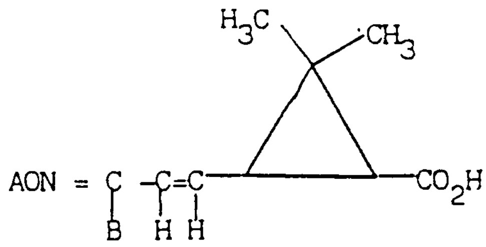

(II)

ihr Säurechlorid oder ihre Anhydride, worin A und B die vorstehend angegebene Bedeutung besitzen, der Einwirkung eines Alkohols der Formel ROH unterzieht, worin R die vorstehend angegebene Bedeutung besitzt, um die entsprechende Verbindung der Formel (I) zu erhalten,und gewünschtenfalls die gegebenenfalls erhaltenen, verschiedenen Stereoisomeren trennt.

**7.** Abänderung des Verfahrens zur Herstellung der Verbindungen der Formel (I) gemäß Anspruch 6, dadurch gekennzeichnet, daß man eine Verbindung der Formel (V)

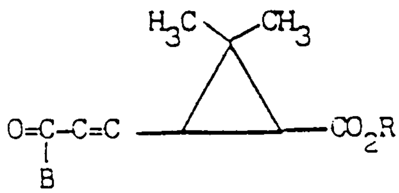

(V)

worin B und R wie in Anspruch 1 definiert sind, der Einwirkung einer Verbindung der Formel (VI)

$AONH_2$ (VI)

unterzieht, worin A die vorstehend angegebene Bedeutung besitzt, um die entsprechende Verbindung der Formel (I) zu erhalten.

**8.** Als Zwischenprodukte die Säuren der Formel (II) sowie ihr Säurechlorid und die Anhydride dieser Säuren, wie in Anspruch 6 definiert.

**9.** Verwendung der Verbindungen der Formel (I), wie in einem der Ansprüche 1 bis 5 definiert, zur Bekämpfung von Parasiten der Pflanzen und Parasiten von Räumlichkeiten.

**10.** Zusammensetzungen für die Bekämpfung von Parasiten der Pflanzen, Parasiten von Räumlichkeiten und Parasiten warmblütiger Tiere, dadurch gekennzeichnet, daß sie als Wirkstoff zumindest eine Verbindung gemäß einem der Ansprüche 1 bis 5 enthalten.

**11.** Insektizide Zusammensetzungen, dadurch gekennzeichnet, daß sie als Wirkstoff zumindest eine Verbindung gemäß einem der Ansprüche 1 bis 5 enthalten.

**12.** Akarizide Zusammensetzungen, dadurch gekennzeichnet, daß sie als Wirkstoff zumindest eine Verbindung gemäß einem der Ansprüche 1 bis 5 enthalten.

**13.** Zusammensetzungen für die tierische Ernährung, dadurch gekennzeichnet, daß sie als Wirkstoff zumindest eine der Verbindungen gemäß einem der Ansprüche 1 bis 5 in Assoziation mit einem tierischen Ernährungsmittel enthalten.

**14.** Assoziationen mit insektizider, akarizider oder nematizider Aktivität, dadurch gekennzeichnet, daß sie als Wirkstoff einesteils zumindest eine der Verbindungen der allgemeinen Formel (I) und anderenteils zumindest einen Pyrethrinoidester enthalten, ausgewählt aus der Gruppe, bestehend aus den Estern der Allethrolone, des 3,4,5,6-Tetrahydrophthalimidomethylalkohols, des 5-Benzyl-3-furylmethylalkohols,

des 3-Phenoxybenzylalkohols und der α-Cyano-3-phenoxybenzylalkohole der Chrysanthemumsäuren, unter den Estern des 5-Benzyl-3-furylmethylalkohols der 2,2-Dimethyl-3-(2-oxo-3-tetrahydrothiophenylidenmethyl)-cyclopropan-1-carbonsäuren, unter den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, unter den Estern der α-Cyano-3-phenoxybenzylalkohole der 2,2-Dimethyl-3-(2,2-dibromvinyl)-cyclopropan-1-carbonsäuren, unter den Estern des 3-Phenoxybenzylalkohols der 2-p-Chlorphenyl-2-isopropylessigsäuren, unter den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tetrahaloethyl)-cyclopropan-1-carbonsäuren, worin "halo" für ein Fluor-, Chlor- oder Bromatom steht, wobei es sich versteht, daß die Verbindungen der Formel (I) in sämtlichen ihrer möglichen stereoisomeren Formen vorliegen können ebenso wie die Säureverknüpfungen und die vorstehenden Alkohole der Pyrethrinoidester.